# EUROPÄISCHE PATENTSCHRIFT

(11) **EP 1 527 742 B1**
(45) Veröffentlichungstag und Bekanntmachung des Hinweises auf die Patenterteilung: **26.07.2006**
(21) Anmeldenummer: 03405786.9
(22) Anmeldetag: 31.10.2003
(51) Int. Cl.: A61B 17/70

(54) **Verschlussvorrichtung für Pedikelschrauben zur Fixierung von elastischen Stabelementen**
Locking cap for pedicle screws for fixing of elastic rods
Dispositif de fermeture de vis p?diculaires pour fixer des tiges de liaison ?lastiques

(43) Veröffentlichungstag der Anmeldung: 04.05.2005
(73) Patentinhaber: Spinelab AG, 6304 Zug (CH)
(72) Erfinder: Freudiger, Stefan, 3047 Bremgarten (CH)
(74) Vertreter: BOVARD AG

(56) Entgegenhaltungen:
- WO-A-95/01132
- WO-A-03/015648
- DE-A- 19 534 136
- DE-A- 19 540 180
- DE-A- 19 951 145
- FR-A- 2 780 269
- US-A1- 2003 125 742

## Beschreibung

Die vorliegende Erfindung betrifft ein Befestigungssystem zur Stabilisierung von Wirbelsäulen gemäss dem Oberbegriff des Patentanspruchs 1. Sie dient dem schnellen, sicheren und einfachen Befestigen und Verbinden während einer Operation in der Wirbelsäulenchirurgie.

Im Stand der Technik gibt es eine grosse Auswahl an Pedikelschrauben mit Verschlusseinrichtungen, die in der Regel einen Aufnahmeteil aufweisen, der zur Aufnahme eines Stabes eine U-förmige Ausnehmung besitzt, die zwischen ihren freien Schenkeln ein Innengewinde für die Aufnahme einer Schraube zur Fixierung des Stabes aufweisen.

US-A5,496,321 beschreibt eine Pedikelschraube mit einem Verankerungsmittel. Dieses Produkt hat jedoch den Nachteil, dass das Verankerungsmittel in Richtung des Stabes in den Schraubenkopf eingefügt werden muss, was bei kleinen Krümmungsradien des Stabes unter Operationsbedingungen zu erheblichen Schwierigkeiten führen kann.

US-A1 -2003/0004511 beschreibt ein Pedikelschraubensystem, dessen Aufnahmeschenkel für einen Stab mit einem Gelenk verbunden sind. Nach der Aufnahme des Stabes werden die Aufnahmeschenkel mit einer Verschlusskappe oder einem Verschlussbügel versehen, welche die Schenkel zusammendrücken und den Stab festhalten. Zum Aufsetzen der Verschlusskappe oder des -bügels müssen die Schenkel des Kopfes der Pedikelschraube zusammengedrückt werden, und gleichzeitig muss das Verschlussstück in der Stabrichtung eingeschoben oder gedreht bzw. der Verschlussbügel eingehängt werden, was nicht nur erhebliches Geschick des Operateurs, sondem je nach Ausführung, noch ein spezielles Instrument erfordert.

DE 199 51 145 A1 offenbart eine Osteosynthesevorrichtung mit einer Pedikelschraube, mit einem eine Nut aufweisenden Gabelkopf und einem in der Nut des Gabelkopfes gelagerten Korrekturstab, wobei der Grund der Nut des Gabelkopfes mit mehreren parallel zueinander liegenden Kerben versehen ist und der Korrekturstab mittels eines Rastdeckels an der Pedikelschraube fixiert ist.

Der vorliegenden Erfindung liegt demzufolge die Aufgabe zugrunde, ein Befestigungssystem zur Stabilisierung von Wirbelsäulen bestehend aus einem elastischen Stab und einer Pedikelschraube mit einer Verschlusseinrichtung zur Verfügung zu stellen, bei welcher die Kraftübertragung vom Stab auf die Schraube auf der Offenseite gewährleistet ist und verhindert wird, dass der Stab nach hinten herausrutschen kann, und die ein sicheres und schnelles Verschliessen der Aufnahme des Stabes in der Pedikelschraube erlaubt, um diesen darin festzuhalten. Weiter muss die Verschlusseinrichtung schnell auch wieder demontierbar sein.

Es wurde gefunden, dass diese Aufgaben gelöst werden können durch eine Verschlusskappe, welche die Funktion einer Traverse ausübt, die am Stab in eine vorhandene Rillenstruktur eingreift und die so aufgenommenen Kräfte in Längsrichtung des Stabs auf die auch mit Rillen versehenen Flanken der offenen Aufnahme der Pedikelschrauben überträgt. Weiterhin weist die Verschlussvorrichtung ein Einrastmittel (z.B. zwei Haken) auf. Hierzu muss die Verschlusskappe elastisch beweglich sein so dass sie beim Einführen in die Aufnahme der Pedikelschraube selbst einrastet (Einhaken). Weiter müssen für ein schnelles und sicheres Wiederentfernen entsprechende Mittel verfügbar sein, wo ein entsprechendes Instrument, vorzugsweise eine Universalinstrument, eingreifen kann.

Gegenstand der vorliegenden Erfindung ist demzufolge das Befestigungssystem gemäss der Definition nach Anspruch 1.

Die abhängigen Ansprüche haben vorteilhafte Weiterbildungen der Erfindung zum Inhalt.

Die Pedikelschraube mit der Verschlussvorrichtung gemäss der Erfindung ist nützlich für das schnelle und sichere Verschliessen und Verbinden eines elastischen Stabelementes mit einer Pedikelschraube mit einer gleichzeitigen Kraftübertragung von der hinteren Stabseite auf die Flanken der Pedikelschraube. Die für die Verschlussvorrichtung vorgesehene Verschlusskappe ist zweckmässigerweise aus einem biokompatiblen Kunststoff hergestellt, vorzugsweise aus Polyurethan der Qualität "medical grade", der sich durch Spritzgiessen verarbeiten lässt. Zum Einführen der Verschlusskappe in das passende Halteelement rasten die an der Verschlusskappe vorgesehen Haken in die passenden Vertiefungen in den Schenkeln der Verschlussvorrichtung am Kopf der Pedikelschrauben selbsttätig ein, sobald sie in der korrekten Position sind. Weiter sind Mittel vorgesehen, die ein einfaches Entfernen dieser Verschlussvorrichtungen ermöglichen, wenn dies während der aktuellen oder einer späteren Operation erforderlich sein sollte. Eine kinematische Umkehrung von Haken und Vertiefungen ist ebenfalls möglich und soll von der Definition der vorliegenden Erfindung umfasst werden, obschon die Geometrie der Vertiefungen angepasst werden muss, was jedoch für einen Fachmann nicht ein unlösbares Problem darstellt.

Mindestens ein, vorzugsweise zwei, in der Aufnahmeausnehmung vorgesehener Einrastvorsprung dient einer Vorfixierung des Stabelements im Aufnahmeelement bevor die Verschlusskappe eingesetzt wird, wobei das Stabelement sowohl teilweise als auch vollständig formschlüssig im Aufnahmeelement gehalten werden kann, derart, dass zur Korrektur der Lage ein leichtes Entfernen und Wiedereinsetzen des Stabelements während einer Operation möglich ist. Hierzu besitzen sowohl das Stabelement als auch die Aufnahmeausnehmung eine ineinander passende Rillen/Rippenstruktur in Querrichtung zum Stabelement. Ein Zusammenspiel des Einrastvorsprungs oder der Einrastvorsprünge mit der erwähnten formschlüssigen Struktur erlaubt ein Vorfixieren in Längs- und Querrichtung zum Stabelement. Hierzu ist die Rillenstruktur im Innem der Aufnahmeausnehmung vorzugsweise bis an die Enden ihrer Schenkel ausgedehnt. Die erwähnten Einrastvorsprünge können so positioniert werden, dass sie im eingerasteten Zustand einen leichten Druck auf das Stabelement ausüben bzw. formschlüssig in den Stab eindringen, wobei auch ein leichter Versatz vorgesehen werden kann. In verschlossenem Zustand ermöglicht die Verschlussvorrichtung mit den genannten Merkmalen ebenfalls die Aufnahme von Torsionskräften, die vom Stabelement ausgeübt werden.

In den beiliegenden Figuren wird die Erfindung anhand von Beispielen erläutert, wobei verschiedene Merkmale anhand ihres Zweckes und ihrer Funktion erklärt werden; es zeigt.
Fig. 1 eine typische Pedikelschraube mit Aufnahmeelement und Verschlusskappe gemäss der Erfindung;
Fig. 2 eine Seitenrissdarstellung der Verschlusskappe in der in Fig. 1 dargestellten Pedikelschraube gemäss der Erfindung;
Fig. 3 einer Oberansicht der der in Fig. 2 dargestellten Verschlusskappe;
Fig. 4 eine weitere Ausführungsform einer Pedikelschraube mit Aufnahmeelement und Verschlusskappe gemäss der Erfindung;
Fig. 5 eine Seitenrissdarstellung der Verschlusskappe in der in Fig. 4 dargestellten Pedikelschraube;
Fig. 6 einer Oberansicht der in Fig. 5 dargestellten Verschlusskappe.
Fig. 7 eine Seitenrissdarstellung einer alternativen Verschlusskappe mit einer S-Schlaufe;
Fig. 8 einer Oberansicht der in Fig. 7 dargestellten Verschlusskappe.

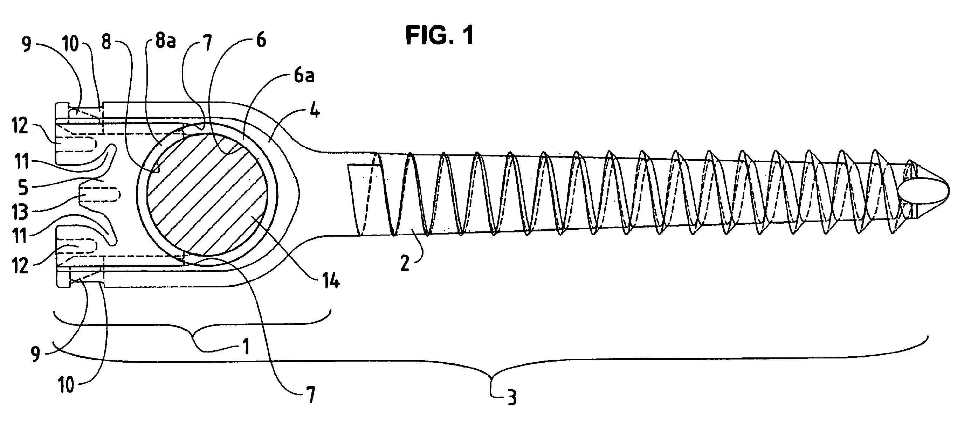

Fig. 1 stellt eine typische Ausführungsform einer erfindungsgemässen Verschlussvorrichtung 1 dar und bildet zusammen mit einem Gewindeteil 2 eine Pedikelschraube 3, wobei die Verschlussvorrichtung deren Kopf darstellt. Das aus zwei Schenkeln bestehende Aufnahmeelement 4 mit seiner U-förmigen Aufnahmeausnehmung 6 dient der Aufnahme eines elastischen Stabelements 14, wobei ein Einrastvorsprung 7 einer Vorfixierung des Stabelements 14 dient, derart, dass das Stabelement erforderlichenfalls während der Operation problemlos von einer an einem Rückenwirbel implantierten Pedikelschraube wieder entfernt werden kann. Das leichte Einrasten wird durch die Elastizität des Stabelements und/oder der U-förmigen Halteausnehmung des Halteelements bestimmt. Das Stabelement 14, die Aufnahmeausnehmung 6 und die Halteausnehmung 8 können eine ineinander passende Struktur aufweisen, z. B. Rippen oder Rillen 8a, 6a, vorzugsweise in Querrichtung zum Stabelement. In der dargestellten Ausführungsform sind die Rillen oder Rippen in der Aufnahmeausnehmung an ihren Schenkeln, zur Optimierung einer Vorfixierung und zur frühzeitigen Längspositionierung, bis nach aussen gezogen. Durch die genannten Merkmale wird eine optimale Kraftübertragung vom Stabelement 14 auf die Pedikelschraube 3 bewirkt, wobei die als Traverse fungierende Verschlusskappe, die nachstehend näher beschrieben wird, mit ihrer Halteausnehmung 8 formschlüssig in das Stabelement 14 eingreift.

Ist die Position des Stabelements korrekt, wird die Verschlusskappe 5 in das Aufnahmeelement 4 eingesetzt. Hierzu dient ein angepasstes Instrument, wie ein Universalschraubendreher, auf welches die Verschlusskappe über die Ausnehmung 13 aufgesetzt werden und die Verschlusskappe in korrekter Position passgenau in die Aufnahmeausnehmung unter selbsttätigem Einrasten eingesetzt werden kann. Zur Entnahme bzw. zur Korrektur wird ein weiteres Instrument verwendet, z.B. eine Universalzange, das Eingreifvorsprünge für die Einführung in die Ausnehmungen 12 aufweist. Die Verschlusskappe besitzt beidseitig Einrastelemente (Haken) 9, die unter Deformation der Verschlusskappe in der Region der Einrastausnehmungen 10 zum Einrasten dienen. Soll die Verschlusskappe 5 wiederum aus dem Aufnahmeelement 1 entfernt werden, wird sie mittels der genannten Universalzange entfernt. Durch das Eingreifen der Zange in die Ausnehmungen 12 und leichtes Zusammendrücken der Zange wird die elastische Verschlusskappe zusammengedrückt, wobei die Einrastelemente 9 aus den Einrastausnehmungen 5 herausgezogen werden und die Verschlusskappe aus der Aufnahmeausnehmung 6 wieder entfernt werden kann.

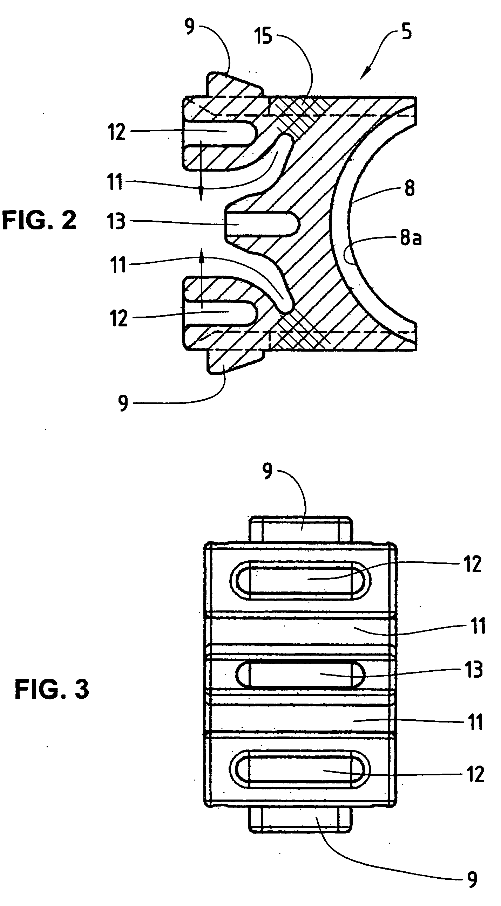

Fig. 2 zeigt eine Schnittzeichnung der Verschlusskappe der in Fig. 1 dargestellten Verschlussvorrichtung gemäss der Erfindung. Die Bezugszeichen entsprechen den für Fig. 1 angegebenen Bedeutungen. Zusätzlich sind die federnden Deformationsbereiche 15 gezeigt, welche ein Zusammendrücken mit dem genannten Spezialwerkzeug in Richtung der Pfeile der Verschlusskappe im oberen Teil erlauben, derart, dass der gegenseitige Abstand der Einrastelemente (Haken) reduziert wird, was ein selbsttätiges Einrasten und auch eine Entnahme der Verschlusskappe aus der Aufnahmeausnehmung durch Ausrasten bewirkt.

Fig. 3 ist eine Ansicht der Verschlusskappe von oben, wobei die Bedeutung der Bezugszeichen wiederum den obigen Definitionen entspricht.

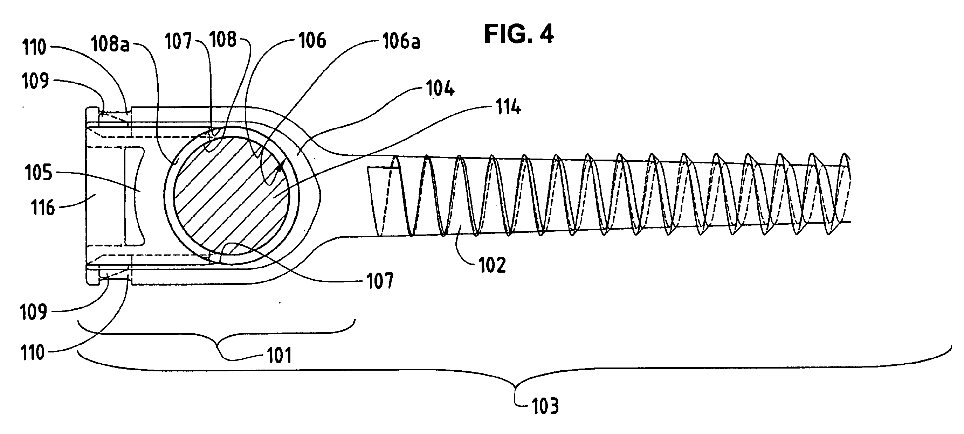

Fig. 4 stellt eine alternative Ausführungsform einer erfindungsgemässen Verschlussvorrichtung 101 dar und bildet zusammen mit einem Gewindeteil 102 eine Pedikelschraube 103. Das Aufnahmeelement 104 dient mit seiner U-förmigen Aufnahmeausnehmung 106 der Aufnahme eines elastischen Stabelements 114, wobei ein Einrastvorsprung 107 einer Vorfixierung des Stabelements 114 dient, derart, dass das Stabelement erforderlichenfalls während der Operation problemlos von einer an einem Rückenwirbel implantierten Pedikelschraube wieder entfernt werden kann. Das Stabelement 114, die Aufnahmeausnehmung 106 und die Halteausnehmung 108 können auch hier eine ineinander passende Struktur aufweisen, z.B. Rippen oder Rillen 106a, 108a, vorzugsweise in Querrichtung.

Ist die Position des Stabelements korrekt, wird die Verschlusskappe 105 in das Aufnahmeelement 104 eingesetzt. Die Verschlusskappe besitzt beidseitig Haken oder Einrastelemente 109, die unter Deformation der Verschlusskappe in der Region der Einrastausnehmungen zum Einrasten in die Einrastausnehmungen 110 des Aufnahmeelements 101 dienen. Ein Beulsteg 116 unterstützt im eingerasteten Zustand den Druck auf die Haken 109 und damit den festen Sitz der Verschlusskappe.

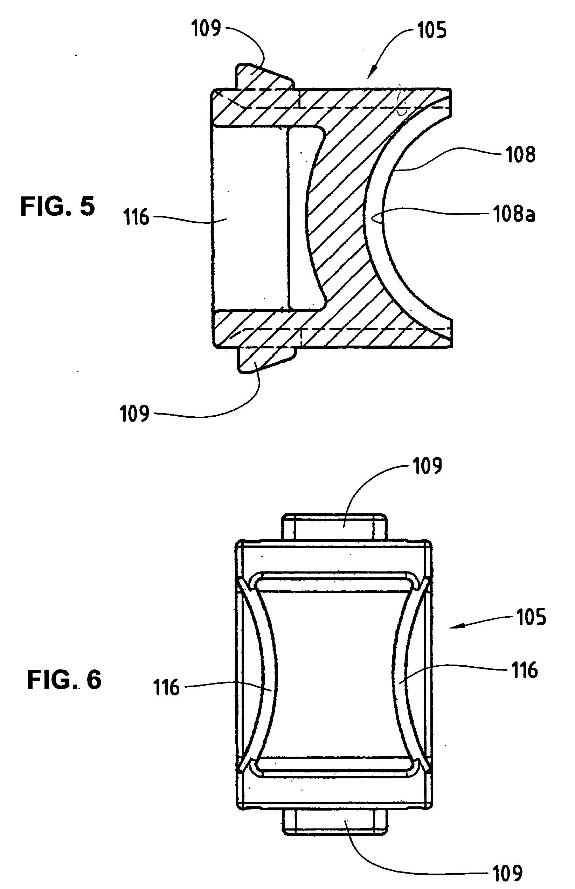

Fig. 5 zeigt eine Schnittzeichnung der Verschlusskappe der in Fig. 4 dargestellten Verschlussvorrichtung gemäss der Erfindung. Die Bezugszeichen entsprechen den für Fig. 4 angegebenen Bedeutungen.

Fig. 6 ist eine Ansicht der Verschlusskappe von oben, wobei die Bedeutung der Bezugszeichen wiederum den obigen Definitionen entspricht.

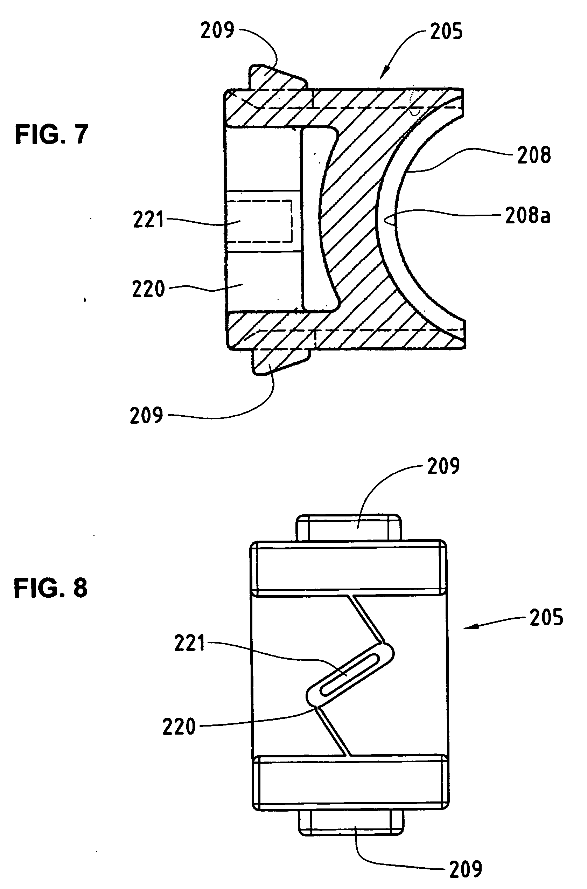

Fig. 7 zeigt eine Schnittzeichnung einer alternativen Verschlusskappe für eine erfindungsgemässe Verschlussvorrichtung. Die Bezugszeichen haben folgende Bedeutungen: 205 die Verschlusskappe; 208 die Halteausnehmung; 208a Rippen/Rillen und 209 die Haken. 220 bedeutet eine S-Schlaufe mit einem Sackloch zum Einführen eines Schraubendrehers, wobei eine Drehbewegung ein Zusammenrücken der Haken 209 und somit eine Freigabe der Verschlusskappe bewirkt.

Fig. 8 ist eine Ansicht der alternativen Verschlusskappe gemäss Fig. 7 von oben, wobei die Bedeutung der Bezugszeichen wiederum den obigen Definitionen entspricht.

## Patentansprüche

1. Befestigungssystem zur Stabilisierung von Wirbelsäulen bestehend aus einem elastischen Stabelement (14) und einer Pedikelschraube (3) mit einer Verschlussvorrichtung (1), umfassend ein Aufnahmeelement (4) mit einer U-förmigen Aufnahmeausnehmung, zwischen welche eine Verschlusskappe (5) zum schnellen Verschliessen des Aufnahmeelements (4) der Pedikelschraube (3) einschiebbar ist, um ein darin einlegbares elastisches Stabelement (14), das der Stabilisierung einer Wirbelsäule dient, aufzunehmen und mit der Verschlussvorrichtung (1) über eine Halterung festzuhalten, **dadurch gekennzeichnet, dass** das Stabelement (14), die U-förmige Aufnahmeausnehmung (6) des Aufnahmeelements (4) und die Verschlusskappe (5) jeweils eine gerippte oder gerillte Oberflächenstruktur aufweisen, die einander entsprechen und die an den jeweiligen Kontaktstellen miteinander in Eingriff kommen, derart, dass die Verschlusskappe die Funktion einer Traverse ausübt und **dadurch** die Kräfte, die in Längsrichtung des Stabelementes (14) wirksam sind, auf die Pedikelschraube (3) übertragbar sind.

2. Pedikelschraube nach Anspruch 1, **dadurch gekennzeichnet, dass** die Halterung aus Einrastmitteln (9), die an der Verschlusskappe angeordnet sind, und aus passenden Aufnahmemitteln in der U-förmigen Aufnahmeausnehmung (4) besteht, wobei die Einrastmittel beim Einsetzen der Verschlusskappe (5) in das Aufnahmeelement selbsttätig einrasten.

3. Pedikelschraube nach Anspruch 2, **dadurch gekennzeichnet, dass** die Einrastmittel als mindestens zwei entgegengesetzt gerichteten relativ zueinander federnd angeordneten Haken (9) ausgestaltet sind, so dass die Verschlusskappe (5) in an den beiden Schenkeln des Aufnahmeelements (4) vorgesehenen Einrastausnehmungen (10) einrasten und im eingesetzten Zustand eine stabile Position einnehmen kann.

4. Pedikelschraube nach Anspruch 3, dadruch **gekennzeichnet**, dass die Verschlusskappe (5) neben den federnd angeordneten Haken (9) mit Vertiefungen (12, 13) bzw. Vorsprüngen versehen ist, die ein Eingreifen eines Instrumentes ermöglichen und damit einerseits mittels einer zentralen Vertiefung (13) ein präzises Einsetzen erzielt und andererseits durch Zusammendrücken von peripheren Vertiefungen (12) oder Vorsprüngen der Abstand der Haken (9) reduziert werden kann, was unter Ausrasten ein Ausfahren der Verschlusskappe (5) erlaubt.

5. Pedikelschraube nach einem der Ansprüche 1 bis 4, **dadurch gekennzeichnet, dass** die gerippte oder gerillte Oberflächenstruktur quer zum Stabelement (14) ausgerichtet ist, und dass in der Aufnahmeausnehmung (6) die Rippen oder Rillen bis an die Enden der durch die Aufnahme gebildeten Schenkel geführt sind.

6. Pedikelschraube nach Anspruch 3 oder 4, **dadurch gekennzeichnet, dass** in der Verschlusskappe der zwischen den zwei entgegengesetzt gerichteten relativ zueinander federnd angeordneten Haken (9) angeordnete Teil als integrierter federnder Teil, wie als Satz von Beulstegen, oder als S-Schlaufe, ausgestaltet ist.

7. Pedikelschraube nach einem der Ansprüche 1 bis 6, **dadurch gekennzeichnet, dass** die Verschlusskappe aus einem elastischen Kunststoff, z.B. aus Polyurethan, besteht.

8. Pedikelschraube nach einem der Ansprüche 1 bis 7, **dadurch gekennzeichnet, dass** in der U-förmigen Aufnahmeausnehmung mindestens ein und bevorzugt zwei Einrastvorsprünge (7) an einer Position vorhanden sind, wo sie ein Einrasten eines eingesetzten Stabelementes ermöglichen, derart dass beim Einsatz eine korrigierbare Vorfixierung mit Torsionshemmung erzielt werden kann.

## Claims

1. Fastening system for stabilising a vertebral column, consisting of an elastic rod element (14) and a pedicle screw (3) with a closure device (1), including a seat element (4) with a U-shaped seat recess, inside which recess a closure cap (5) is insertable for quick closure of the seat element (4) of the pedicle screw (3), to receive an elastic rod element (14), placeable therein, which serves to stabilize a vertebral column, and to hold it with the closure device (1) via a holder, **characterised in that** the rod element (14), the U-shaped seat recess (6) of the seat element (4) and the closure cap (5) each have a ridged or grooved surface structure, corresponding to one another, and which surface structures come into engagement with one another at the respective places of contact such that the closure cap carries out the function of a cross-brace, and the forces which are active in lengthwise direction of the rod element (14) are thereby transmittable to the pedicle screw (3).

2. Pedicle screw according to claim 1, **characterised in that** the holder consists of engagement means (9), which are disposed on the closure cap, and of matching receiving means in the U-shaped seat recess(4), the engagement means automatically locking into place during placement of the closure cap (5) in the seat element.

3. Pedicle screw according to claim 2, **characterised in that** the engagement means are designed as at least two oppositely directed hooks (9), resiliently disposed relative to one another, so that the closure cap (5) is able to engage in engagement recesses (10) provided on the two arms of the seat element (4) and to assume a stable position in the inserted state.

4. Pedicle screw according to claim 3, **characterised in that** the closure cap (5) is provided with depressions (12, 13) or respectively projections next to the resiliently disposed hooks (9), which depressions or respectively projections make possible an engagement of an instrument, and a precise insertion can be achieved, on the one hand, by means of a central depression (13), and, on the other hand, the spacing apart of the hooks (9) can be reduced through the pressing together of peripheral depressions (12) or projections, which, upon disengagement, allows a moving out of the closure cap (5).

5. Pedicle screw according to one of the claims 1 to 4, **characterised in that** the ridged or grooved surface structure is aligned transversely to the rod element (14), and **in that** the ridges or grooves in the seat recess (6) are extended until the ends of the arms formed by the seat.

6. Pedicle screw according to claim 3 or 4, **characterised in that** in the closure cap the part disposed between the two oppositely directed hooks (9), resiliently disposed with respect to one another, is designed as an integrated resilient part, such as a set of buckled crosspieces or as S-loops.

7. Pedicle screw according to one of the claims 1 to 6, **characterised in that** the closure cap is composed of an elastic synthetic material, e.g. of polyurethane.

8. Pedicle screw according to claim 1 to 7, **characterised in that** at least one, and preferably two, engagement projections (7) are provided in the U-shaped seat recess at a position where they make possible an engagement of an inserted rod element in such a way that, upon insertion, a correctable pre-fixing with torsion blocking can be achieved.

## Revendications

1. Système de fixation pour la stabilisation de colonnes vertébrales se composant d'un élément en forme de tige élastique (14) et d'une vis pédiculaire (3) avec un dispositif de fermeture (1), comprenant un élément de logement (4) avec un évidement de logement en forme de U entre lesquelles peut être inséré un bouchon de fermeture (5) pour la fermeture rapide de l'élément de logement (4) de la vis pédiculaire (3), pour loger un élément en forme de tige élastique insérable (14), qui sert à la stabilisation d'une colonne vertébrale et de la maintenir par une fixation au dispositif de fermeture (1), **caractérisé en ce que** l'élément formant tige (14), l'évidement de logement en forme de U (6) de l'élément de logement (4) et le bouchon de fermeture (5) présentent respectivement une structure de surface nervurée ou rainurée qui correspondent entre elles de sorte que le bouchon de fermeture exécute la fonction d'une traverse et de ce fait les forces agissant dans le sens longitudinal de l'élément formant tige (14) étant transférable à la vis pédiculaire (3).

2. Vis pédiculaire selon la revendication 1, **caractérisée en ce que** la fixation se compose de moyens d'encliquetage (9), qui sont disposés sur le bouchon de fermeture, et de moyens de logement appropriés dans l'évidement de logement (4) en forme de U, les moyens d'encliquetage s'encliquetant automatiquement lors de l'insertion du bouchon de fermeture (5) dans l'élément de logement.

3. Vis pédiculaire selon la revendication 2, **caractérisée en ce que** les moyens d'encliquetage sont configurés comme au moins deux crochets (9) disposés élastiques et dirigés en opposé l'un par rapport à l'autre, de sorte que le bouchon de fermeture (5) s'encliquète dans des évidements d'encliquetage (10) prévus sur les deux branches de l'élément de logement (4) et peut prendre une position stable à l'état inséré.

4. Vis pédiculaire selon la revendication 3, **caractérisée en ce qu'**outre les crochets disposés élastiquement (9), le bouchon de fermeture (5) est muni de cavités (12, 13) respectivement de saillies, qui permettent l'introduction d'un instrument et il est ainsi possible d'une part au moyen d'une cavité centrale (13) une insertion précise et d'autre part par pression de cavités périphériques (12) ou des saillies de réduire l'écart des crochets (9) ce qui permet par un désencliquetage une sortie du bouchon de fermeture (5).

5. Vis pédiculaire selon l'une des revendications 1 à 4, **caractérisée en ce que** la structure nervurée ou rainurée est orientée transversalement à l'élément de tige (14) et **en ce que** dans l'évidement de logement (6), les rainures ou les nervures sont guidées jusqu'aux extrémités de la branche formée par le logement.

6. Vis pédiculaire selon la revendication 3 ou 4, **caractérisé en ce qu'**une partie disposée entre les deux crochets (9) disposés élastiquement l'un par rapport à l'autre dirigés en opposition mutuelle est ménagée dans le bouchon de fermeture en tant que partie élastique intégrée comme jeu de pattes bombées ou de boucle S.

7. Vis pédiculaires selon l'une des revendications 1 à 6, **caractérisée en ce que** le bouchon de fermeture se compose de matière synthétique élastique par exemple en polyuréthane.

8. Vis pédiculaire selon l'une des revendications 1 à 7, **caractérisée en ce que** dans l'évidement de logement en forme de U au moins une de préférence deux saillies d'encliquetage (7) sont présentes à une position, où elles permettent un encliquetage d'un élément inséré formant tige de sorte qu'à l'usage, une préfixation corrigeable avec blocage de torsion peut être obtenue.
